# EUROPEAN PATENT SPECIFICATION

(11) **EP 1 357 863 B1**
(45) Date of publication and mention of the grant of the patent: **10.11.2010**
(21) Application number: 02720747.1
(22) Date of filing: 02.01.2002
(51) Int. Cl.: A61L 27/46, A61L 27/48, A61L 27/56, A61L 27/58, B29C 47/06, A61F 2/36

(54) **COMPOSITIONS AND METHODS FOR BIOMEDICAL APPLICATIONS**
ZUSAMMENSETZUNGEN UND VERFAHREN FÜR BIOMEDIZINISCHE ANWENDUNGEN
COMPOSITIONS ET PROCEDES DESTINES A DES APPLICATIONS BIOMEDICALES

(30) Priority: 02.01.2001 US 259348 P; 05.11.2001 US 337577 P
(43) Date of publication of application: 05.11.2003
(73) Proprietor: Advanced Ceramics Research, Inc., Tucson, AZ 85706-5013 (US)
(72) Inventor: VAIDYANATHAN, K., Ranji, Tucson, AZ 85715 (US); WALISH, Joseph, Miami, FL 85539 (US); CALVERT, Paul, D., Tucson, AZ 85718 (US)
(74) Representative: Harrison, Ivor Stanley
(86) International application number: PCT/US2002/000051
(87) International publication number: WO 2002/053105

(56) References cited:
- EP-A- 0 192 068
- DE-A1- 19 614 421
- DE-U- 8 812 075
- US-A- 5 766 618
- US-A- 5 955 529
- US-A- 6 027 742

## Description

### FIELD OF THE INVENTION

The present invention relates to biocompatible implants for surgical implantation in bone replacement and bone substitution treatment, particularly with load-bearing applications such as spinal implants.

### BACKGROUND OF INVENTION

As the life expectancy of human beings has increased, so has the need for repair and replacement of bone structures within a body. Implants made from metals, such as titanium alloys, are known. Although such implants are strong, their use presents many problems. Typically, such implants must be fixed into the surrounding bone. Because the frictional properties of the metal differ from those of human bone, the bone will eventually wear away at points of contact with the metal implant, causing a debilitating condition called osteolysis. Additionally, the body may reject the implant As a result of these and other circumstances, only about 80% of orthopedic replacements remain viable after ten years, and the remaining 20% of implants must be removed within ten years or less. The need for additional surgery to US6027742 describes a composite implantable bone replacement material comprising a strongly resorbable, poorly crystalline apatitic calcium phosphate and a biocompatible supplemental material that either is well resorbable or poorly resorbable. DE19614421 describes a porous implantable bone replacement material comprising a calcium phosphate material, a biodegradable polymer such as polylactic acid, and a pore-forming agent providing a porosity between 10% and 60%, the material being formed into rods, ground and pressed to form foils. EP0192068 describes a porous implantable bone replacement material comprising 25% to 75% of a calcium phosphate material, such as tricalcium phosphate; 25% to 75% of a biodegradable polymer such as polylactic acid; and up to 30% by weight of a pore forming agent such as NaCl which can be leached out with water. US3789029 describes a bone prosthesis formed by curing PMMA with grated anorganic bone in a mould to form a shaped implant having sufficient porosity to enable it to become firmly interlocked with surrounding tissue such as bones and membranes. EP0357155 describes a bone implant formed from HPOE (hydantoin polyoxyethylene) and PBT having a porosity of 50% and a pore diameter of 96 micron, showing good tissue ingrowth and bone bonding on implantation in rats. US2001/051832 describes bone implants formed from a polyethylene glycol terephthalate and polybutylene terephthalate copolymer, treated to have 50% porosity and pores of size 100 to 500 microns. EP1027897 describes a porous biodegradable matrix as a cartilage scaffold formed from a copolymer of polyethylene glycol terephthalate/polybutylene terephthalate copolymer prepared by salt leeching or sintering, the matrix being coated with calcium phosphate to provide a ceramic part to mimic the function of the bone, the polymeric part mimicking the function of cartilage. The porosity is preferably between 50% and 95%, the pores preferably being from 1 to 1000 µm in diameter.

### SUMMARY OF THE INVENTION

In one aspect, the present invention provides a biocompatible implant for surgical implantation, the implant comprising; a biocompatible implant for surgical implantation comprising: a matrix comprising a resorbable composition selected from polymethylmethacrylate, polybutyleneterephthalate, polyethyletherketone and combinations thereof, the matrix having a pore size between 100 to 2400 µm and porosity between 25% to 70% by volume, being effective for enhancing bone growth adjacent the composition; **characterised in that** the implant includes a growth-enhancing composition for stimulating new tissue growth at the site of implantation, the growth-enhancing composition including a biocompatible polymer and a calcium source, and in that the resorbable composition degrades upon implantation at a first rate to provide load-bearing support for a predetermined period of time and the growth-enhancing composition degrades upon implantation at a second rate faster than the first rate to stimulate new tissue growth on the implant.

In another aspect, the invention provides a method of fabricating a biomedical implant, the method comprising the steps of: forming a feedrod from a polymer composition selected from polymethylmethacrylate, polybutyleneterephthalate, polyethyletherktone and combinations thereof; passing a first amount of feedrod through a dispensing head and onto a working surface in a predetermined pattern to form a first layer of the polymer composition on the surface; passing a second amount of the feedrod through the dispensing head and onto the previously-formed first layer in a predetermined pattern to form a multilayer object having a pore size between 100 to 2400 µm and porosity between 25% to 70% by volume; characterised by applying onto the multilayer object a growth-enhancing composition for stimulating new tissue growth at the site of implantation and to provide a porous implant object, the growth-enhancing composition including a biocompatible polymer and a calcium source, in which the multilayer object degrades upon implantation at a first rate to provide load-bearing support for a predetermined period of time and the growth-enhancing composition degrades upon implantation at a second rate faster than the first rate to stimulate new tissue growth on the implant.

The present invention also provides the use of a polymer matrix selected from a polymer matrix selected from polymethylmethacrylate, polybutyleneterephthalate, polyethyletherketone and combinations thereof as a resorbable composition and a growth-enhancing composition including a biocompatible polymer selected from polylactic acid, polyglycolic acid, polylactic acid-polyglycolic acid copolymer, polycaprolactone, and combinations thereof, and a calcium source, in the preparation of a biocompatible implant for surgical implantation, the matrix having a pore size between 100 to 2400 µm and porosity between 25% to 70% by volume, the implant being effective for enhancing new growth of bone and tissue for surgically implanting in vivo at a desired site of repair to provide a foundation for new bone and tissue growth, in which the resorbable composition degrades upon implantation at a first rate to provide load-bearing support for a predetermined period of time and the growth-enhancing composition degrades at a second rate faster than the first rate to stimulate new tissue growth on the implant.

The present invention provides compositions and methods for making structures that are suitable for use as medical implants in bone reconstruction and replacement treatment. In important embodiments, the compositions and structures include a bio-compatible, polymeric material or blend of sufficient strength and durability to provide mechanical support of surrounding bone structure for a desired and adequate length of time. The structures are, however, porous, non-permanent and degrade at a controlled rate, allowing bone cells to grow into them until they are substantially replaced by natural bone and tissue. The compositions and structures include a coating or cells of growth-enhancing composition for stimulating and enhancing bone growth and vascularization at the implant site. The growth-enhancing composition degrades at a faster rate than the implant structure so as to provide nutritional and other components that will initially stimulate growth of bone and tissue at the site of the implant.

The compositions and structures can be used in the treatment of fractured bones and joints and in the treatment of degenerative diseases, such as continuous gradual joint damage or Chondromalacia Patella, which is a type of degenerative disease of the kneecap. Other possible applications for the compositions and structures of the present invention include treatment of discogenic disease where a surgical graft is placed in spinal disc space to make it stiffer and help cause fusion and relieve associated pain, metastatic tumor treatment where a short or long segment fusion is surgically performed after removing a large tumor, and infection treatment where a long or short segment fusion is surgically performed and a synthetic graft is used to support the fusion and limit the spread of infection.

In representative embodiments of the invention, the compositions include polymer and polymeric composite materials including a calcium source, thermoplastic materials, co-polymers and combinations thereof. More particularly, such materials include polymethylmethacrylate (PMMA), polybutylene-terephthalate (PBT), and polyethyletherketone (PEEK), and combinations thereof. The biocompatible polymer compositions include polymers, such as polycaprolactone and polylactic-polyglycolic acid, and a calcium source, such as calcium phosphate or calcium sulfate. The growth enhancing compositions also can include additives, such as growth factors, including transforming growth factor β (TGFβ). Preferably, the biocompatible polymer is polycaprolactone and the calcium source is tricalcium phosphate.

The invention also provides methods of making the implants. The compositions are formed into the implant structures using ribbon or filament deposition, or a rapid prototyping process. In comparison to conventional prosthesis manufacturing methods, such as investment casting and machining, rapid prototyping processes allow implants of complex shape, including a porous construction, to be custom-made quickly and relatively easily. The processes allow the implants to be prepared on site and custom fitted to the patient's injury. Such processes also can allow the patient's own osteoblasts and bone morphogenic proteins to be incorporated into the implants, thus enhancing the ability of the implant to readily bond with the trauma zone. The implants can be made in a layer-wise fashion by the sequential stacking of discrete raw material layers upon each other until the desired body part is formed. Each layer has a geometry corresponding to a cross section of the desired implant.

Thus, it is an object of the present invention to provide compositions and materials that are biocompatible and capable of stimulating regrowth of natural bone and tissue, as well as bioresorbable and replaceable by natural bone and tissue.

A further object of the invention is to provide a biomedical implant device that will stimulate the regrowth of natural bone and tissue and provide load-bearing support at the site of implantation until such time as the natural bone and tissue is capable of providing such support.

Yet another object of the invention is to provide methods of making and using the compositions and materials embodying the characteristics set forth above.

These and other objects, advantages, and features of the invention are set forth in the detailed description that follows.

### BRIEF DESCRIPTION OF THE DRAWINGS

FIG. 1 is a schematic of a cross-sectional end view of a structure in accordance with the present invention;
FIG. 2 is a schematic of a portion of a second structure in accordance with the present invention;
FIG. 3 is a schematic of a portion of a third structure in accordance with the present invention;
FIG. 4 is a block diagram illustrating the steps in the manufacture and use of compositions and materials for repair and replacement of tissue in accordance with the present invention;
FIG. 5 is a graph illustrating the relationship between porosity and road width in making the structures of the present invention;
FIG. 6 is a graph illustrating the degradation of a coating composition in accordance with the present invention;
FIG. 7 is a graph illustrating load versus displacement for a structure in accordance with the present invention;
FIG. 8 is a graph illustrating load versus displacement for another structure in accordance with the present invention;
FIG. 9 is a graph illustrating calcium release from a coating composition in accordance with the present invention;
FIG. 10 is a graph illustrating cell growth on a structure in accordance with the present invention;
FIG. 11 is a photomicrograph showing cell growth on a structure in accordance with the present invention; and
FIG. 12 is a graph illustrating alkaline phosphatase activity for a structure in accordance with the present invention.

### DETAILED DESCRIPTION OF THE INVENTION

The present invention relates to compositions and structures formed from such compositions having osteoconductive and/or osteoinductive characteristics thereby enabling use as biomedical implants for bone substitution and replacement in orthopedic and other reconstructive surgical applications. The biocompatible implants include polymer, ceramic or thermoplastic materials and combinations thereof capable of providing a strong support framework within the body, even under load-bearing conditions, for an extended period of time. In addition to providing mechanical support for the surrounding bone structure, the implants also protect natural growing bone as the natural bone and tissue begins to grow at the site of implantation. The implant structures have a predetermined pore size and porosity effective for promoting natural bone growth around the exterior of the implants, as well as within the pore space of the implant.

Preferably, the implant structures of the present invention are non-permanent but provide a strong load-bearing support structure at the site of surgical implantation for a time sufficient to allow the surrounding bone to grow at the site and become load bearing. Thus, the implant structures are resorbed, degraded, eroded or otherwise dissolved and diminished in size over a period of time so that they eventually will be at least substantially replaced by natural bone structure and tissue. As the size of the structures decreases, new growth of the person's natural bone gradually replaces the structures and takes over the load-bearing functions of the implant structures. Degradation of the implant structures occurs at a desired, controlled rate. Preferably, the rate of degradation is generally slow, so that the implant will remain structurally viable at the site of implantation for about one year or more.

As used herein, the term "osteoinductive" means stimulating bone growth and/or vascularization, such as by providing a source of nutrition and enhancing the rate and degree of growth for bone or tissue through the addition of growth enhancing factors such as TGFβ.

As used herein, the term "osteoconductive" means acting as a substrate for bone growth and/or vascularization, or otherwise being conducive to bone growth and/or vascularization. For example, a porous body having sufficient pore space and acting as a substrate on which natural bone can grow, as compared to a solid body, is "osteoconductive." It is understood that the rate of bone growth generally is higher for an osteoinductive material compared to an osteoconductive material.

The composition used to form an implant structure is selected to be mechanically and biologically compatible with the characteristics and functions of the site of implantation. The composition used to form an implant structure preferably is also bioresorbable to allow natural bone to grow and replace the implant structure over time, is osteoinductive and/or osteoconductive to promote bone growth and vascularization, and is biocompatible with tissues and bone structures present at the implant site. The implant structure preferably has suitable surface chemistry for cell attachment, proliferation and differentiation and is sufficiently porous with an interconnected pore network to promote cell growth. The mechanical properties of the implant structure are similar to or compatible with those of tissues at the site of the implant to limit frictional wear.

The compositions for the implant structures of the present invention include thermoplastic materials, such as polymer and/or ceramic composite materials. Preferably, such materials include polymethylmethacrylate (PMMA), polybutylene-terephthalate (PBT), and polyethyletherketone (PEEK), and combinations thereof. Polyglycolic acid-polylactic acid copolymers and tricalcium phosphate also may be used as porous scaffold materials for enhancing bone growth, however, they alone generally do not possess the mechanical properties needed for a load-bearing bone scaffold or substrate.

The selection of biomaterials for these applications plays a key role in the design and development of tissue engineering product development. While the classical selection criterion for a safe, stable bioimplant dictated choosing a passive, "inert" material, it is now understood that any such material will generally elicit a cellular response that is not necessarily desirable. Therefore, it is now considered important in product design to choose a biomaterial that acts to predispose tissue to repair rather than act as a replacement. Thus, biomaterials or combinations thereof used in tissue repair and replacement must not only be biocompatible but must elicit a desirable cellular response, as well. Consequently, controlling and manipulating the cellular interactions with biomaterials is a significant goal, especially for tissue engineering applications.

The implant compositions also can include processing aids, such as surfactants, compatibilizers and fillers, or other desired additives. Examples of surfactants and compatibilizers include polyethylene glycol (PEG) or methoxy polyethylene glycol (MPEG) and the like. These additives can assist in coating the polymer surfaces and can reduce the loads required to extrude them during implant structure fabrication. Examples of fillers include calcium sulfate (Franklin Fiber) and the like which are used to improve strength further by aligning themselves in the direction of extrusion. Another filler that can be used is poly-2-ethyl-2-oxazoline (PEOx), a water-soluble polymer. By dissolving the PEOx after fabrication of implant structures, an additional level of porosity may be provided to the implant materials or structure to assist in vascularization.

A biodegradable, biocompatible polymeric composition can be incorporated into or applied onto the surface of the implant structures to further enhance the rate and degree of bone growth and vascularization. Preferably, the growth-enhancing composition is applied at or near the surface of the implant structure and coats substantially the entire surface area of the implant structure, as well as fills the pore space of the structure. The growth-enhancing composition begins to degrade after the implant structure is surgically implanted, thereby releasing inorganic solutes, such as calcium and phosphate, at a controlled rate to enhance bone growth. As the natural bone grows in and around the implant, the bone utilizes the minerals and nutritional supplements released by the growth-enhancing composition for further growth.

The growth-enhancing composition begins to dissolve, degrade or erode after implantation, and the bone begins to grow at the site. The growth-enhancing composition stimulates initial bone growth. The bone eventually replaces the composition within the pore space and on the surface of the implant structure. Preferably, the growth-enhancing composition will remain on the implant structure for up to no more than about three months. In comparison, the implant composition (e.g., PMMA, PEEK, PBT, etc.) degrades at a slower rate than the growth-enhancing composition so as to generally maintain its integrity during the initial phase of bone growth to provide load-bearing support for the surrounding bone structure and protection for the new bone and tissue. The implant composition and structure is only subsequently replaced by growing bone after the initial period when the bone begins to grow and form a bone framework at the implant site. Thus, the gradual resorption of the implant composition allows secondary bone formations to be established and bone remodeling to take place in a stable fashion by load transfer to the ingrown tissue.

The ceramic, growth-enhancing composition used to impregnate the pore space includes polymers, such as polycaprolactone or copolymers of polylactic acid and polyglycolic acid, or linear aliphatic polyesters such as polylactic acid and polyglycolic acid. The composition also includes a calcium source, such as calcium phosphate or calcium sulfate. Preferably, the polymer is polycaprolactone and the calcium source is tricalcium phosphate. The polymers and calcium source are blended together at ratios of between about 1:1 to about 1:5, preferably between about 1:1.5 to about 1:2.5, and more preferably about 1:2, to ensure that the viscosity of the blend is between about 100-500 centipoise (CPS) at about 80-100°C. An advantage with compositions containing polycaprolactone is that the viscosity of the blend is as low as about 100-400 CPS at temperatures between about 80-100°C.

The composition also can include additives, such as those for enhancing bone and tissue regrowth and for improving the biocompatibility of the implant structure with the host body. The composition also can include pharmaceutical additives, such as antibiotics, analgesics, antiinflammatories, hormones, immunosuppressants, and the like. Suitable bioactive additives include growth factors such as bone morphogenic protein (BMP), transforming growth factor (alpha, TGFα, and beta, TGFβ), and platelet derived growth factor, osteogenic growth factors such as bone-derived growth factor, activin, insulin-like growth factor, basic fibroblast growth factor and combinations thereof. An additive that enhances tissue regrowth such as TGFβ is particularly preferred. The composition also can include ceramic materials such as hydroxy apatite and the like.

The growth-enhancing composition is applied to the outer surface of the implant structure, preferably after the structure is formed, although it may also be blended into the polymer substrate during the deposition process when the structures are formed. The growth-enhancing composition preferably is heated to enhance flowability of the composition during application to allow uniform coating. Preferably, the composition is heated to about 80°C to about 100°C. The composition is applied to the structure so that it coats the surface of the implant and substantially fills the pore space. Mylar or similar sheet-like material can be placed on the implant and a vacuum applied so that the mylar collapses around the porous structure to maintain the growth-enhancing composition as a generally uniform coating on the surface of the structure and within the porous interior. Upon cooling, the composition solidifies and remains on the surface of the structure and within the pores.

In another embodiment, the growth-enhancing coating is applied as a water-based suspension. The polymer and calcium phosphate of the composition are blended in a water-based latex solution that also includes a surfactant. The latex solution is densified by heating. The growth-enhancing composition is then applied to the structure to form a coating on the surface and within the pore space of the structure.

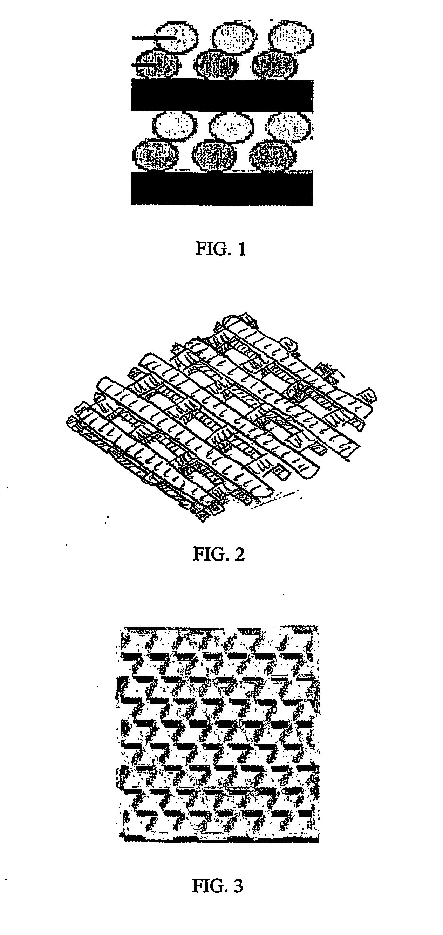

The structures can be formed in any configuration, shape and size suitable for the particular implant application. For example, flat plates or discs may be desired for a particular application, while three-dimensional shapes of various geometries such as conical, frustoconical, kidney, spherical and tubular shapes may be desired for others. Generally, the structure is formed to have a desired porosity and pore size, preferably to provide a foundation that facilitates cellular organization and tissue regeneration to promote regrowth of tissue and bone at the site of implantation. A porous structure presents a favorable surface for cell attachment and growth, thereby enhancing the implant's ability to serve as a biodegradable scaffold for tissue repair or implant fixation. During formation, ribbons or filaments of the composition are extruded in layers onto a work or support surface with the ribbons or filaments generally being deposited layer upon layer parallel to the work or support surface. The individual layers of ribbon can be deposited in any desired arrangement, with adjacent layers having the ribbons arranged at various angles to the adjacent layers. For example, as illustrated in FIGS. 1-4, configurations such as off-set (FIG. 1), scaffold, where filaments of each layer are arranged at 90° angles relative to filaments in adjacent layers, (FIG. 2), and lattice, where the filaments of each layer are arranged at angles of less than 90° relative to filaments in adjacent layers, (FTG. 3). A preferred configuration is a scaffold configuration (FIG. 2). Other shapes and configurations also are contemplated so long as the desired characteristics, including porosity and pore size, and the desired functions, including protecting growing bone and providing mechanical support to the surrounding bone structure, are achieved. Preferably, the edges of the structure are rounded to promote and stimulate bone growth at the implant

The implant structures can be made using modified ribbon or filament deposition process, such as an extrusion freeform (EFF) process for forming three-dimensional bodies. EFF processes that may be modified for use in making the structures of the present invention are known, for example those described in U.S. Patent Nos. 5,340,433; 5,121,329; 5,932,290; 6,070,107. An EFF process can be adapted to allow for rapid fabrication of functional components from the compositions of the present invention. The process allows for the sequential deposition of multiple layers of the compositions to form complex-shaped structures as desired. Preferably, the EFF process equipment includes a fabrication modeler fitted with a high-pressure extrusion head to allow for extrusion of the highly viscous polymer systems of the present invention. Generally, a pre-formed feedrod is prepared from the composition and passed to EFF apparatus. Alternatively, the components of the composition, including polymer materials and additives, can be passed directly to the apparatus for a continuous process. The feedrod is passed through an extrusion apparatus where an extrusion head of the apparatus deposits the extruded composition onto a work or support surface. The ribbons or filaments of extruded material generally are deposited layer upon layer onto the work or support surface in a predetermined pattern to form an object of the desired shape and size and having the desired porosity characteristics. Preferably, the extruded material is deposited so that the longitudinal axis of the extruded material is generally parallel to the work surface.

The compositions can be formed into structures of various shapes and sizes, including geometrically complex objects, having the desired pore size and porosity. Functionally graded and hierarchical composites can be co-deposited using an EFF process. Thus, using a modified EFF process, high-strength thermoplastic and thermoset polymer composite structures having enhanced mechanical properties can be extruded into the structures of the present invention for use in surgical implant applications. In addition, such a process allows for the extrusion of much higher viscosity feedstock compared to other traditional freeform fabrication techniques.

Using a modified EFF process or other suitable technique, the compositions of the present invention are formed into structures having microchannels containing polymer filler components to approximate, for example, natural bone structures. Porosity parameters (e.g., pore size, structure and distribution) of the implant structures can be selected to optimize growth of natural bone and tissue into the implant. Additionally, the implant can include more than one portion, each exhibiting one or more different parameters, for example to permit different cell ingrowth into the implant to occur at different rates. The structures have connected pore structures with pores sizes between about 100 to about 2400 µm and a porosity level of between about 25 to about 70%. Larger and smaller pores can be formed in the structure by varying the placement of the ribbon of implant composition. It is also contemplated that structures having pore size distributions that are mono-modal, bi-modal or polymodal are within the scope of the invention. Preferably, the structures have pore sizes between about 150 to about 400µm and a porosity level of between about 50 to about 60% by volume. As the pore size decreases, the ease and effectiveness of application of the growth-enhancing composition is reduced. The initial degree of porosity of the implant structures should be such that the implant structure is capable of substantially maintaining its structural integrity for a desired period of time, even as the implant structure begins to degrade over time.

Once formed, the implant structures can be further processed as desired to provide a finished implant structure. After application of the composition, the structures can be heated to a temperature and for a time sufficient to anneal the structures to reduce the residual stresses created during fabrication of the structure. Annealing provides structures having enhanced flexural strength and higher flexural modulus as compared to structures that are not annealed. The structures also can be cleaned to achieve a reasonably good surface smoothness. Additionally, the implant structures can tooled or otherwise shaped to obtain the final desired implant shape.

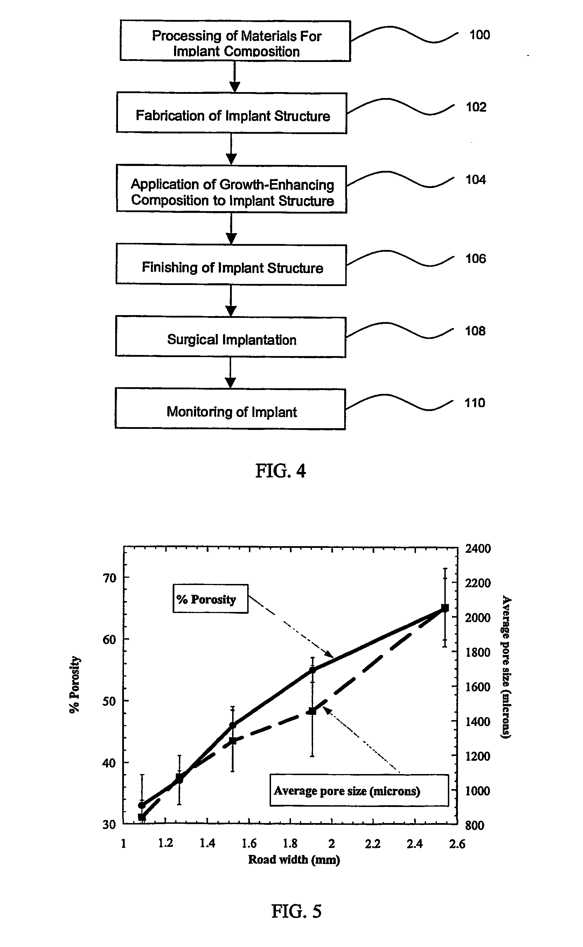

The invention provides methods of making implant structures and of repairing a tissue site using the compositions and materials described herein. According to such methods, as generally illustrated in FIG. 4, the materials of the implant compositions are processed and blended 100. The composition is formed into an implant structure 102 of desired size, shape and porosity using an automated technique, such as the extrusion freeform fabrication process described above. Once the implant structure is formed, a growth-enhancing composition is applied to the structure 104. The composition can be dried or adhered to the implant structure as described above. The coated implant structure then is ready for final processing 106. Such processing can include annealing, cleaning and tooling. The implant structure then is surgically implanted *in vivo* at the implant site 108 and monitored 110 to ensure that the implant is accepted by the body host and that a desired rate and degree of bone and tissue regrowth is achieved.

### EXAMPLES

The following examples are intended to illustrate the present invention and should not be construed as in any way limiting or restricting the scope of the present invention.

### Example 1

Experiments were conducted regarding the formulation of the polymer compositions for forming the implant structures. Poly-2-ethyl-2-oxazoline (PEOx) was mixed with PBT and calcium phosphate. The blending was performed at 215°C. Typical free-formable PEOx/PBT blend combinations are given in Table 1. Experiments indicated blending could be performed at 215°C even though the melting point of PBT was 250°C. Feedrods of the blend were made and extruded with the extrusion freeform fabrication ("EFF") process. However, since the blending temperature was much lower than the melting point of the PBT material, small chunks of PBT remained in the blend. Therefore, the material did not extrude effectively during freeform fabrication. Consequently, it is believed that blending at a higher temperature will provide complete blending of the PBT with the PEOx. It was noted that the addition of any acid containing groups to PEOx tended to degrade the blends by breaking down the PEOx, especially if the blends remained in the hot zone of the Brabender mixer. If the PBT blends contain even small amounts of acid groups, the PEOx may be broken down into individual monomers which leads to an increase in the torque during mixing.

**Table 1**

| Component | Concentration (Vol%) |
|---|---|
| Poly-2-ethyl-2-oxazoline | 36 |
| PBT | 46 |
| Calcium phosphate | 10 |
| Compatibilizer/plasticizer | 8 |

### Example 2

Experiments were conducted to optimize the EFF operating parameters. Critical variables were: start delay, main flow, roll back, speed and road width (e.g., final width of the extruded ribbon of material upon cooling). Table 2 shows the optimized values obtained for the EFF process using a 0.0016" size nozzle tip. These values will vary slightly with other nozzle tip sizes. The extrusion temperature in each case will depend on the material being extruded.

Porous test samples of PMMA and PBT were fabricated using these process conditions. A series of 1" diameter PMMA test samples were fabricated with raster road widths varying from 1.09 mm (0.0429") to 2.54 mm (0.1"). In previous experiments, it was observed that the nominal raster road width obtained for a 0.41 mm (0.016") tip was 0.64 to 0.76 mm. That is, although the material is extruded in a ribbon of substantially circular cross-section, upon deposition the material will settle somewhat to form a ribbon having, for example, a generally oval cross-sectional shape of dimensions that are wider but shorter than the diameter of the initial ribbon. Therefore, to create a porous sample, a larger raster road width was set in the commercially available software that created the motion architecture program. For example, for a nominally 30% porosity sample, the raster road width was proportionally increased from 0.76 mm, which would be the road width obtained for a dense sample. Thus, a raster road width of 1.09 mm (0.0429") would provide a 70% dense or 30% porous sample, based on the ratio of nominal road width and the road width set in the commercially available software. Extrusion was intentionally varied between 155 and 160°C.

**Table 2**

| Parameter | Optimized EFF process value |
|---|---|
| Start delay | 0.82 sec |
| Preflow | 79 |
| Start flow | 89 |
| Start distance | 0.06" |
| Main flow | 260% |
| Shut off distance | 0.073" |
| Rollback | 229 |
| Speed | 0.293"/sec |
| Acceleration | 5 |

Porosity levels were calculated for each sample, based on the sample's dimensions and weight, and the density of PMMA. The average pore sizes were measured for each specimen at 50x magnification using a optical microscope. The relationship between the road width, % porosity, and the average pore size is shown in Table 3 and plotted in FIG. 5. Both the % porosity and the average pore sizes of the test pieces increased with increasing road width. Porosity levels can be manipulated by modifying the extrusion temperature. However, the effect of road width is greater than the effect of extrusion temperature on the average porosity levels.

**Table 3**

| Road width (mm) | % Porosity | Average pore size (µm) |
|---|---|---|
| 1.09 | 33 ± 5 | 838 ± 99 |
| 1.27 | 37 ± 4 | 1067 ± 37 |
| 1.52 | 46 ± 3 | 1278 ± 177 |
| 1.91 | 55 ± 2 | 1453 ± 262 |
| 2.54 | 65 ± 5 | 2053 ± 227 |

Initially, problems occurred with the fabrication of PBT specimens due to delamination. However, increasing the envelope temperature inside the EFF build envelope to 55°C, which is below the glass transition temperature of PBT, overcame the delamination problem. Testing indicated that porosity percentage and average pore size of porous test specimens of any material can be accurately controlled during extrusion freeforming. Therefore, a sample requiring a specific pore size and porosity percentage can be obtained based on the optimum pore size required for effective impregnation. Typically, average pore sizes of at least about 150-4.00 µm and porosity levels of about 50% by volume are needed for effective impregnation.

The extrusion tip size was changed to 0.0012" to obtain small pore sizes and high porosity levels. Average pore sizes of about 150-250 µm and porosity levels of about 50-60% were obtained with 0.0012" extrusion tip. Samples having 15 mm diameters were created using the 0.0012" extrusion tip. A much finer distribution of pore sizes can be obtained than otherwise possible with a 0.0016" extrusion tip size.

### Example 3

Tests were performed to determine the effectiveness of impregnating a porous specimen with an osteoinductive polymer/ceramic blend. For example, polycarbonate specimens having a series of through holes were tested. The specimens were impregnated with blend consisting of polycaprolactone (Tone-polyol 0260 from Union Carbide) and polycaprolactone mixed with 3β calcium phosphate. Polycaprolactone is a biocompatible liquid, however, other biocompatible liquids, such as copolymers of 50:50 polylactic-polyglycolic acid may also be used.

The blends were heated to about 80-100°C to enable an easy flow. The specimens were impregnated with the heated blends and covered with a thin sheet of mylar. A vacuum was applied on the specimens so that the mylar sheet collapsed around the polycarbonate sheet and held the impregnated blend in place. On cooling, the blend solidified and stayed inside the pores.

Using the above technique, several more porous test specimens were impregnated with polymer/ceramic blends. All samples were polished with sand paper, cleaned in acetone, surrounded with the polymer-calcium phosphate mixtures, covered in mylar film, and placed inside an oven at about 80-100°C. The ratio of polycaprolactone and 3 β calcium phosphate was about 1:1. After impregnation, the samples were cleaned to achieve a reasonably good surface smoothness.

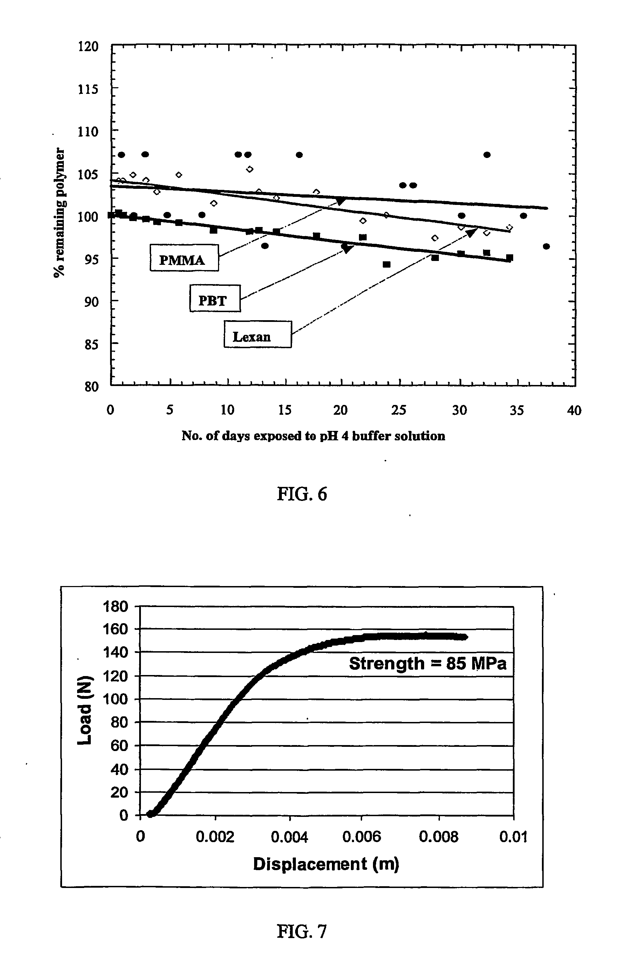

The degradation properties of these materials in the impregnated condition were studied in neutral and acidic simulated body fluids. Samples were then exposed to buffer solutions of pH 7, pH 4 and pH 3. The sample weights were monitored as a function of time over a period of approximately five weeks. No appreciable weight loss was observed on any of the polymer materials after exposure to the buffer solutions. Tables 4-6 show the weight change versus time of exposure to a pH 4 solution for samples made from lexan (a form of polycarbonate), PBT and PMMA respectively. These samples were impregnated with about 1:1 polycaprolactone:calcium phosphate polymer-ceramic mixture. The rate of weight loss can be expressed as a linear function of time. This data is also shown in FIG. 6 which shows that the weight loss after five weeks of exposure was minimal for each of the polymers. Similar results were obtained after exposure to a buffer solution with pH = 3.

**Table 4**

| Time of exposure (mins) | Total Weight (g) | Remaining Polymer (g) | % remaining |
|---|---|---|---|
| 0.00E+00 | 2.49 | 0.15 | 100.00 |
| 8.87E+02 | 2.493 | 0.153 | 102.00 |
| 1.45E+03 | 2.492 | 0.152 | 101.33 |
| 2.65E+03 | 2.492 | 0.152 | 101.33 |
| 4.18E+03 | 2.492 | 0.152 | 101.33 |
| 5.52E+03 | 2.491 | 0.151 | 100.67 |
| 8.17E+03 | 2.49 | 0.15 | 100.00 |
| 1.26E+04 | 2.49 | 0.15 | 100.00 |
| 1.70E+04 | 2.49 | 0.15 | 100.00 |
| 1.82E+04 | 2.489 | 0.149 | 99.33 |
| 2.04E+04 | 2.486 | 0.146 | 97.33 |
| 2.55E+04 | 2.484 | 0.144 | 96.00 |
| 3.14E+04 | 2.483 | 0.143 | 95.33 |
| 3.43E+04 | 2.472 | 0.132 | 88.00 |
| 3.43E+04 | 2.472 | 0.132 | 88.00 |
| 4.01E+04 | 2.481 | 0.141 | 94.00 |
| 4.32E+04 | 2.48 | 0.14 | 93.33 |
| 4.64E+04 | 2.48 | 0.14 | 93.33 |
| 4.93E+04 | 2.478 | 0.138 | 92.00 |

**Table 5**

| Time of exposure (mins) | Total Weight (g) | Remaining Polymer (g) | % remaining |
|---|---|---|---|
| 0.00E+00 | 2.05 | 1.007 | 100.00 |
| 8.87E+02 | 2.053 | 1.01 | 100.30 |
| 1.45E+03 | 2.05 | 1.007 | 100.00 |
| 2.65E+03 | 2.047 | 1.004 | 99.70 |
| 4.18E+03 | 2.045 | 1.002 | 99.50 |
| 5.52E+03 | 2.042 | 0.999 | 99.21 |
| 8.17E+03 | 2.041 | 0.998 | 99.11 |
| 1.26E+04 | 2.032 | 0.989 | 98.21 |
| 1.70E+04 | 2.031 | 0.988 | 98.11 |
| 1.82E+04 | 2.032 | 0.989 | 98.21 |
| 2.04E+04 | 2.031 | 0.988 | 98.11 |
| 2.55E+04 | 2.026 | 0.983 | 97.62 |
| 3.14E+04 | 2.024 | 0.981 | 97.42 |
| 3.43E+04 | 1.992 | 0.949 | 94.24 |
| 3.43E+04 | 1.992 | 0.949 | 94.24 |
| 4.01E+04 | 2 | 0.957 | 95.03 |
| 4.32E+04 | 2.005 | 0.962 | 95.53 |
| 4.64E+04 | 2.006 | 0.963 | 95.63 |
| 4.93E+04 | 2.001 | 0.958 | 95.13 |

**Table 6**

| Time of exposure (mins) | Total Weight (g) | Remaining Polymer (g) | % remaining |
|---|---|---|---|
| 0.00E+00 | 0.384 | 0.028 | 100.00 |
| 1.20E+03 | 0.386 | 0.03 | 107.14 |
| 2.73E+03 | 0.384 | 0.028 | 100.00 |
| 4.07E+03 | 0.386 | 0.03 | 107.14 |
| 6.72E+03 | 0.384 | 0.028 | 100.00 |
| 1.11E+04 | 0.384 | 0.028 | 100.00 |
| 1.56E+04 | 0.386 | 0.03 | 107.14 |
| 1.67E+04 | 0.386 | 0.03 | 107.14 |
| 1.89E+04 | 0.383 | 0.027 | 96.43 |
| 2.32E+04 | 0.386 | 0.03 | 107.14 |
| 2.91E+04 | 0.383 | 0.027 | 96.43 |
| 3.63E+04 | 0.385 | 0.029 | 103.57 |
| 3.75E+04 | 0.385 | 0.029 | 103.57 |
| 4.32E+04 | 0.384 | 0.028 | 100.00 |
| 4.64E+04 | 0.386 | 0.03 | 107.14 |
| 5.11E+04 | 0.384 | 0.028 | 100.00 |
| 5.39E+04 | 0.383 | 0.027 | 96.43 |

By increasing the acidity of the buffer solution or the calcium phosphate ratio in the biopolymer mixture, the rate of polymer degradation can be increased (Table 7 and Table 8).

An impregnated PBT sample was observed after exposure to a pH 4 buffer solution for two weeks. An impregnated PMMA sample was observed after exposure to a pH 4 solution for two weeks. No visual loss of strength or damage to these samples after exposure to the buffer solutions was indicated. Additionally, initial cell growth studies suggested that these materials degrade by hydrolytic surface erosion. However, the rate of surface erosion is highly dependent on the percentage of calcium phosphate, the acidity level of the buffer solution as well as the pore size and porosity of the specimens.

**Table 7**

| Time of exposure (mins) | Total Weight (g) | Remaining Polymer (g) | % remaining |
|---|---|---|---|
| 0.00E+00 | 2.662 | 0.136 | 100.00 |
| 4.46E+03 | 2.668 | 0.142 | 104.41 |
| 5.63E+03 | 2.666 | 0.14 | 102.94 |
| 7.79E+03 | 2.663 | 0.137 | 100.74 |
| 1.30E+04 | 2.66 | 0.134 | 98.53 |
| 1.89E+04 | 2.656 | 0.13 | 95.59 |
| 3.47E+04 | 2.658 | 0.132 | 97.06 |
| 4.05E+04 | 2.656 | 0.13 | 95.59 |
| 4.36E+04 | 2.655 | 0.129 | 94.85 |
| 4.68E+04 | 2.655 | 0.129 | 94.85 |
| 4.97E+04 | 2.654 | 0.128 | 94.12 |

**Table 8**

| Time of exposure (mins) | Total Weight (g) | Remaining Polymer (g) | % remaining |
|---|---|---|---|
| 0.00E+00 | 0.855 | 0.422 | 100.00 |
| 3.20E+03 | 0.853 | 0.42 | 99.53 |
| 6.06E+03 | 0.848 | 0.415 | 98.34 |

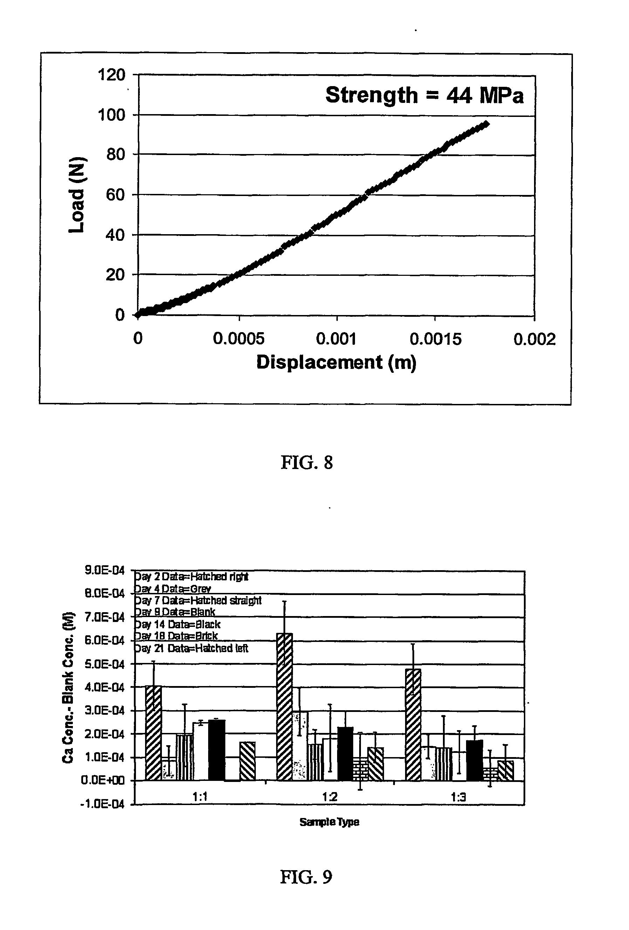

The mechanical strength of PBT and PMMA samples was measured in 3-pt flexure. For PBT samples, the strength and flexural modulus were 81 ± 6 MPa and 4320 MPa respectively. This is higher than a 50:50 PLA:PGA, which is a conventional material, that has a strength and modulus of 50 MPa and 3000 MPa. A typical load-displacement curve for the rapid-prototyped PBT material is shown in FIG. 7. The strength and modulus of the rapid-prototyped PMMA material were 44 ± 1 MPa and 1730 ± 49 Mpa, which is significantly lower than for PBT. A typical load-displacement curve for the rapid-prototyped PMMA material is shown in FIG. 8.

### Example 4

Invitro calcium dissolution studies were performed on impregnated pre-forms having constant weight and area (15 mm diameter). The samples were impregnated with the polycaprolactone-calcium phosphate composite mixtures in a 1:1 ratio. The dissolution media was sterile filtered 0.05M TRIS (pH 7.4) and 0.05M MES (pH 5.5) containing 0.1% sodium azide.

The samples were incubated at 37°C in 20 ml of the buffer solutions. The solutions were changed every other day and both the pH and the Ca potentials were recorded. There were no morphological changes observed in the specimens measured for calcium release.

In a second study, samples were sterilized using ethylene oxide prior to the calcium release and bone cell behavior studies. The calcium release rates from the first set of specimens that were impregnated with the 1:1 PCL:TCP during 1 week were low compared to rates reported for polylactic acid. Similar studies were conducted with specimens that were impregnated with 1:2 and 1:3 PCL:TCPcompositions. With higher TCP contents in the impregnation compositions, it was observed that calcium release rates were improved considerably. This is shown in FIG. 9. It can be seen that the calcium release rates were highest in the first two days. However, the calcium release rates were considerably lower with the 1:3 PCL:TCP mixtures. The calcium release rates and the cell growth were lower for these samples because of the presence of traces of the solvent used to assist the impregnation of the highly viscous 1:3 PCL:TCP mixtures. The calcium release rate results was also confirmed with the cell growth study results. Calcium release rates can be improved by using either polylactic acid or by reducing the polycaprolactone content in the mixture.

### Example 5

*In vitro* tests for bone cell behavior were performed on flat circular implants (15 mm diameter) similar in configuration to the structure of FIG. 2. Rat post-natal calvaria was isolated, dissected and cells digested in a collagenase solution. Cells were grown to confluence in complete DMEM under standard sterile conditions, and used after the second passage. The bone cells were isolated and cultured in the implants. During testing, about 100,000 cells were added to each type of implant and tissue culture plastic. After two days, the media was changed and on the third day, an MTT assay was performed, which determines the cell growth. These results are shown in Table 9.

**Table 9**

| Type | Number of cells |
|---|---|
| Cell culture plates | 525, 000 (avg. of n=6) |
| 100-125 µm PBT 15 mm discs | 149, 400 (n = 1) |
| 150-200 µm PBT 15 mm discs | 198, 200 (n = 1) |

The cells did not die or decrease in concentration, but rather, increased in concentration in three days. The cell concentration was more on the 150-200 µm implant sample. The 100-125 µm sample had more debris when the implant sample was taken off the culture plates. This study indicates that PBT implants are biocompatible.

### Example 6

Circular PBT specimens (15mm in diameter and 4mm thick) similar in configuration to the structure of FIG. 2 were tested to evaluate the growth-enhancing compositions. The specimens had an average porosity size of 150-200) µm and were coated with a continuous layer of tricalcium phosphate (TCP) in a polycaprolactone (PCL) binder. The specimens tested were (i) virgin (U), (ii) vacuum impregnated with TCP-PCL mixture (Vi), and (iii) latex coated with TCP-PCL mixture (Lc). The latex coating was a water-based suspension of TCP and PCL latex densified by heat treatment. This latex method is solvent-free and allows the internal structure of the scaffold to be uniformly coated. Rat post-natal calvaria was isolated, dissected and cells digested in a collagenase solution. Cells were grown to confluence in complete DMEM under standard sterile conditions, and used after the second passage. Flat ethylene oxide sterilized circular implants were seeded with 5x10⁵ calvarial cells in 24 well tissue culture plates. Each of the implants and tissue culture plate surfaces (TP) were assayed in triplicate for 2, 7, and 14 days for alkaline phosphatase activity (APA) and cell growth. APA was determined by a colorimetric assay following trypsin removal of cells from the implants and wells with p-nitrophenolphosphate. Cell growth was assayed by an MTT colorimetric assay (Sigma Kit # CGD-1). Seeded and unseeded implants were viewed by SEM. The mean and standard errors were calculated and significances determined at p<0.05 by ANOVA and post-hoc multiple range tests.

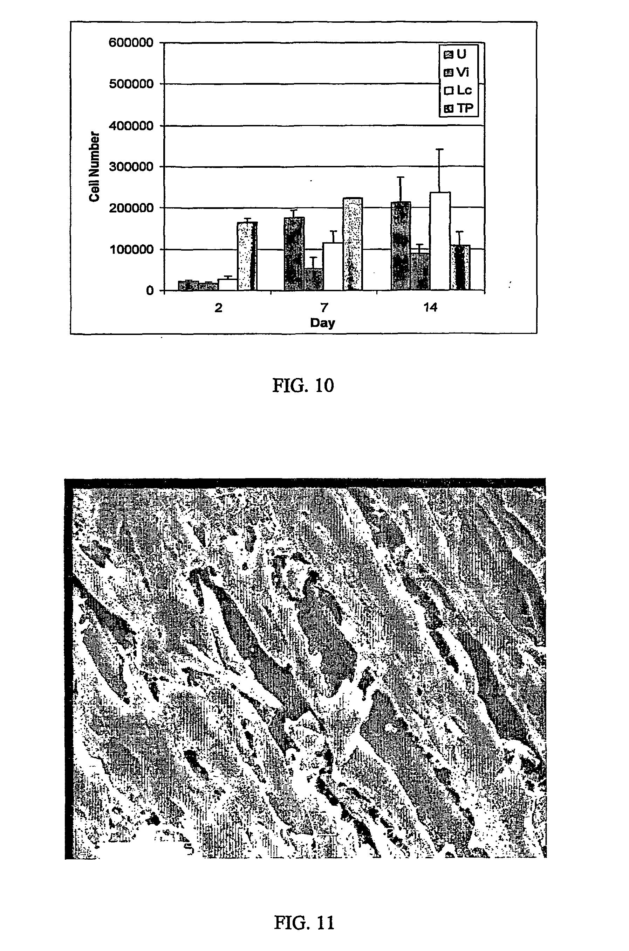

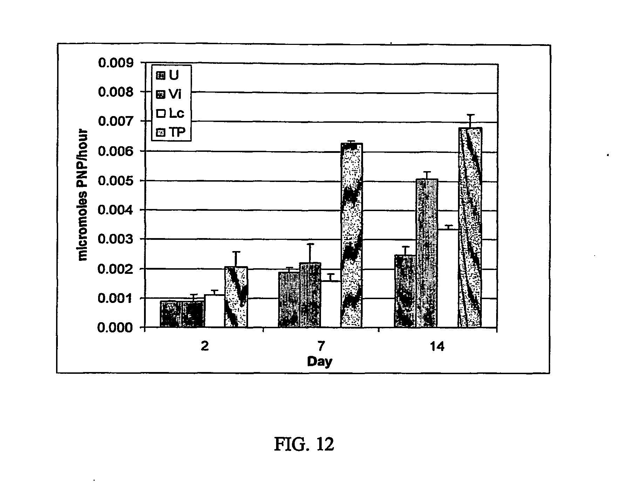

Scanning electron microscopy (SEM) of the latex coated scaffolds showed a uniform coating. SEMs of cell-seeded scaffolds demonstrated cellular in-growth. Cell morphology demonstrated enhanced affinity to the latex coated scaffolds. At 7 and 14 days *in vitro,* Lc scaffolds exhibited a higher degree of cell growth compared to the Vi scaffolds (FTG. 10), and reached values for cell growth of U scaffolds at 14 days. The SEM image of the cell growth on a Vi scaffold impregnated with a 1:2 PCL:TCP mixture is shown in Fig. 11. Cell APA was greater on Vi than Lc and U scaffolds (FIG. 12).

## Claims

1. A biocompatible implant for surgical implantation comprising:
a matrix comprising a resorbable composition selected from polymethylmethacrylate, polybutyleneterephthalate, polyethyletherketone and combinations thereof, the matrix having a pore size between 100 to 2400 µm and porosity between 25% to 70% by volume, being effective for enhancing bone growth adjacent the composition; **characterised in that** the implant includes
a growth-enhancing composition for stimulating new tissue growth at the site of implantation, the growth-enhancing composition including a biocompatible polymer and a calcium source, and **in that** the resorbable composition degrades upon implantation at a first rate to provide load-bearing support for a predetermined period of time and the growth-enhancing composition degrades upon implantation at a second rate faster than the first rate to stimulate new tissue growth on the implant.

2. An implant according to claim 1, wherein in use the natural bone structure substantially replaces the implant after a predetermined time.

3. An implant according to claim 1 or claim 2, wherein the growth-enhancing composition is applied on the surface of the implant as a coating over substantially the entire surface area of the implant and fills the pore space thereof.

4. An implant according to any of claims 1 to 3, wherein the growth-enhancing composition further comprises one or more transforming growth factors.

5. An implant according to any of claims 1 to 4, wherein the biocompatible polymer is selected from polycaprolactone, copolymers of polylactic acid and polyglyolic acid, linear aliphatic polyesters, and blends thereof.

6. An implant according to any of claims 1 to 5, wherein the growth-enhancing composition is blended with the resorbable composition.

7. An implant according to any of claims 1 to 6, wherein the calcium source is calcium phosphate.

8. A method of fabricating a biomedical implant comprising the steps of:
forming a feedrod from a polymer composition selected from polymethylmethacrylate, polybutyleneterephthalate, polyethyletherktone and combinations thereof;
passing a first amount of feedrod through a dispensing head and onto a working surface in a predetermined pattern to form a first layer of the polymer composition on the surface;
passing a second amount of the feedrod through the dispensing head and onto the previously-formed first layer in a predetermined pattern to form a multilayer object having a pore size between 100 to 2400 µm and porosity between 25% to 70% by volume; **characterised by**
applying onto the multilayer object a growth-enhancing composition for stimulating new tissue growth at the site of implantation and to provide a porous implant object, the growth-enhancing composition including a biocompatible polymer and a calcium source, in which the multilayer object degrades upon implantation at a first rate to provide load-bearing support for a predetermined period of time and the growth-enhancing composition degrades upon implantation at a second rate faster than the first rate to stimulate new tissue growth on the implant.

9. A method according to claim 8, wherein the porous implant object is heated for a time and at a temperature effective for annealing the object.

10. A method according to claim 8 or claim 9, wherein a thin, flexible material is wrapped around the porous implant object and a vacuum applied to provide an outer covering for holding the biocompatible composition on the multilayer object.

11. A method according to any of claims 8 to 10, wherein the biocomptible polymer is selected from polylactic acid, polyglycolic acid, polylactic acid-polyglycolic acid copolymer, polycaprolactone, and combinations thereof.

12. A method according to any of claims 8 to 11, wherein the biocompatible polymer and the calcium source are blended at ratios of between 1:1 to 1:5.

13. A method according to any of claims 8 to 12, wherein the viscosity of the polymer composition is between 100 to 500 centipoise at temperatures between 80° to 100°C.

14. The use of a polymer matrix selected from polymethylmethacrylate, polybutyleneterephthalate, polyethyletherketone and combinations thereof as a resorbable composition and a growth-enhancing composition including a biocompatible polymer selected from polylactic acid, polyglycolic acid, polylactic acid-polyglycolic acid copolymer, polycaprolactone, and combinations thereof, and a calcium source, in the preparation of a biocompatible implant for surgical implantation, the matrix having a pore size between 100 to 2400 µm and porosity between 25% to 70% by volume, the implant being effective for enhancing new growth of bone and tissue for surgically implanting in vivo at a desired site of repair to provide a foundation for new bone and tissue growth, in which the resorbable composition degrades upon implantation at a first rate to provide load-bearing support for a predetermined period of time and the growth-enhancing composition degrades at a second rate faster than the first rate to stimulate new tissue growth on the implant.

## Patentansprüche

1. Biokompatibles Implantat zur operativen Implantation umfassend:
eine Matrix umfassend eine resorbierbare Zusammensetzung, die aus Polymethylmethacrylat, Polybutylenterephthalat, Polyethyletherketon und Kombinationen derselben ausgewählt ist, wobei die Matrix eine Porengröße zwischen 100 bis 2400 µm und eine Porosität zwischen 25 Vol.-% bis 70 Vol.-% aufweist, und wirksam ist bei der Förderung des Knochenwachstums neben der Zusammensetzung, **dadurch gekennzeichnet, dass** das Implantat
eine wachstumsfördernde Zusammensetzung zur Stimulierung von neuem Gewebewachstum an der Implantationsstelle beinhaltet, die wachstumsfördernde Zusammensetzung ein biokompatibles Polymer und eine Calciumquelle beinhaltet und dass die resorbierbare Zusammensetzung bei Implantation mit einer ersten Geschwindigkeit abgebaut wird, um für eine vorgegebene Zeitdauer eine lasttragende Unterstützung bereit zu stellen, und die wachstumsfördernde Zusammensetzung bei Implantation mit einer zweiten Geschwindigkeit abgebaut wird, die größer als die erste Geschwindigkeit ist, um das neue Gewebewachstum auf dem Implantat zu stimulieren.

2. Implantat nach Anspruch 1, bei dessen Anwendung die natürliche Knochenstruktur das Implantat nach einer vorgegebenen Zeitdauer im wesentlichen ersetzt.

3. Implantat nach Anspruch 1 oder Anspruch 2, wobei die wachstumsfördernde Zusammensetzung auf die Oberfläche des Implantats als ein Beschichtung über im wesentlichen die gesamte Oberfläche des Implantats aufgetragen wird und den Porenraum desselben füllt.

4. Implantat nach einem der Ansprüche 1 bis 3, wobei die wachstumsfördernde Zusammensetzung darüber hinaus einen oder mehrere transformierende(n) Wachstumsfaktor(en) umfasst.

5. Implantat nach einem der Ansprüche 1 bis 4, wobei das biokompatible Polymer aus Polycaprolakton, Copolymeren aus Polymilchsäure und Polyglykolsäure, linearen aliphatischen Polyestern und Mischungen derselben ausgewählt ist.

6. Implantat nach einem der Ansprüche 1 bis 5, wobei die wachstumsfördernde Zusammensetzung mit der resorbierbaren Zusammensetzung gemischt wird.

7. Implantat nach einem der Ansprüche 1 bis 6, wobei es sich bei der Calciumquelle um Calciumphosphat handelt.

8. Verfahren zur Fertigung eines biomedizinischen Implantats umfassend die Schritte:
Bilden eines Zuführstabs aus einer Polymerzusammensetzung, die aus Polymethylmethacrylat, Polybutylenterephthalat, Polyethyletherketon und Kombinationen derselben ausgewählt ist,
Durchleiten einer ersten Menge des Zuführstabs durch einen Spenderkopf und auf eine Arbeitsoberfläche in einem vorgegebenen Muster, um eine erste Schicht der Polymerzusammensetzung auf der Oberfläche zu bilden,
Durchleiten einer zweiten Menge des Zuführstabs durch den Spenderkopf und auf die zuvor gebildete erste Schicht in einem vorgegebenen Muster, um ein mehrschichtiges Objekt mit einer Porengröße zwischen 100 bis 2400 µm und einer Porosität zwischen 25 Vol.-% bis 70 Vol.-% zu bilden,
**gekennzeichnet durch**
Auftragen einer wachstumsfördernden Zusammensetzung auf das mehrschichtige Objekt zur Stimulierung von neuem Gewebewachstum an der Implantationsstelle und zur Bereitstellung eines porösen Implantatobjekts, wobei die wachstumsfördernde Zusammensetzung ein biokompatibles Polymer und eine Calciumquelle beinhaltet, wobei das mehrschichtige Objekt bei Implantation mit einer ersten Geschwindigkeit abgebaut wird, um für eine vorgegebene Zeitdauer eine lasttragende Unterstützung bereit zu stellen und die wachstumsfördernde Zusammensetzung bei Implantation mit einer zweiten Geschwindigkeit abgebaut wird, die größer als die erste Geschwindigkeit ist, um das neue Gewebewachstum auf dem Implantat zu stimulieren.

9. Verfahren nach Anspruch 8, wobei das poröse Implantatobjekt für eine Zeitdauer und bei einer Temperatur, die zum Ausheilen des Objekts wirksam sind, erhitzt wird.

10. Verfahren nach Anspruch 8 oder Anspruch 9, wobei ein dünnes, flexibles Material um das poröse Implantatobjekt herum gewickelt wird und ein Vakuum angelegt wird, um eine äußere Abdeckung bereit zu stellen, um die biokompatible Zusammensetzung auf dem mehrschichtigen Objekt zu halten.

11. Verfahren nach einem der Ansprüche 8 bis 10, wobei das biokompatible Polymer aus Polymilchsäure, Polyglykolsäure, Polymilchsäure-Polyglykolsäure-Copolymer, Polycaprolakton und Kombinationen derselben ausgewählt ist.

12. Verfahren nach einem der Ansprüche 8 bis 11, wobei das biokompatible Polymer und die Calciumquelle in Verhältnissen zwischen 1:1 bis 1:5 vermischt sind.

13. Verfahren nach einem der Ansprüche 8 bis 12, wobei die Viskosität der Polymerzusammensetzung bei Temperaturen zwischen 80° bis 100° zwischen 100 bis 500 Centipoise beträgt.

14. Anwendung einer Polymermatrix, die aus Polymethylmethacrylat, Polybutylenterephthalat, Polyethyletherketon und Kombinationen derselben ausgewählt ist, als eine resorbierbare Zusammensetzung und einer wachstumsfördernden Zusammensetzung, die ein biokompatibles Polymer beinhaltet, das aus Polymilchsäure, Polyglykolsäure, Polymilchsäure-Polyglykolsäure-Copolymer, Polycaprolakton und Kombinationen derselben ausgewählt ist, und einer Calciumquelle bei der Herstellung eines biokompatiblen Implantats zur operativen Implantation, wobei die Matrix eine Porengröße zwischen 100 bis 2400 µm und eine Porosität zwischen 25 Vol.-% bis 70 Vol.-% aufweist, das Implantat wirksam ist bei der Förderung des neuen Wachstums von Knochen und Gewebe zur operativen Implantation unter Invivo-Bedingungen an einer gewünschten Reparaturstelle, um eine Grundlage für neues Wachstum von Knochen und Gewebe bereit zu stellen, wobei die resorbierbare Zusammensetzung bei Implantation mit einer ersten Geschwindigkeit abgebaut wird, um für eine vorgegebene Zeitdauer eine lasttragende Unterstützung bereit zu stellen, und die wachstumsfördernde Zusammensetzung mit einer zweiten Geschwindigkeit abgebaut wird, die größer als die erste Geschwindigkeit ist, um neues Gewebewachstum auf dem Implantat zu stimulieren.

## Revendications

1. Implant biocompatible pour l'implantation chirurgicale comprenant :
- une matrice renfermant une composition résorbable choisie parmi le polyméthyleméthacrylate, le polybutylènetéréphthalate, la polyéthyléthercétone et leurs combinaisons, cette matrice ayant une dimension de pores comprise entre 100 et 240 µm et une porosité comprise entre 25 % et 70 % en volume, et étant apte à activer la croissance osseuse au voisinage de la composition,
**caractérisé en ce que**
l'implant comporte :
- une composition activant la croissance pour stimuler la croissance de nouveau tissu sur le site d'implantation, cette composition renfermant un polymère biocompatible et une source de calcium, et **en ce que** la composition résorbable se décompose dés l'implantation à une première vitesse pour former une ossature support pendant une période de temps prédéfinie, tandis que la composition activant la croissance se décompose dés l'implantation à une seconde vitesse supérieure à la première vitesse pour stimuler la croissance de nouveau tissu sur l'implant.

2. Implant selon la revendication 1,
dans lequel
à l'usage, la structure de l'os naturel remplace essentiellement l'implant après une durée prédéfinie.

3. Implant selon la revendication 1 ou 2,
dans lequel
la composition activant la croissance est appliquée sur la surface de l'implant, sous la forme d'un revêtement, sur essentiellement la totalité de la surface de l'implant et remplit le volume de ses pores.

4. Implant selon l'une des revendications 1 à 3,
dans lequel
la composition activant la croissance renferme en outre au moins un facteur de croissance transformant.

5. Implant selon l'une des revendications 1 à 4,
dans lequel
le polymère biocompatible est choisi parmi la caprolactone, les copolymères d'acide polylactique et d'acide polyglyolique, les polyesters aliphatiques linéaires et leurs mélanges.

6. Implant selon l'une des revendications 1 à 5,
dans lequel
la composition activant la croissance est mélangée à la composition résorbable.

7. Implant selon l'une des revendications 1 à 6,
dans lequel
la source de calcium est du phosphate de calcium.

8. Procédé d'obtention d'un implant biomédical comprenant les étapes consistant à :
- former une baguette d'alimentation à partir d'une composition polymère choisie parmi le polyméthyleméthacrylate, le polybutylènetéréphthalate, la polyéthyléthercétone et leurs combinaisons,
- faire passer une première quantité de cette baguette d'alimentation au travers d'une tête de distribution et sur une surface de travail selon un motif prédéterminé pour former une première couche du polymère sur la surface,
- faire passer une seconde quantité de la baguette d'alimentation au travers de la tête de distribution et sur la première couche préalablement formée avec un motif prédéfini pour former un objet multicouches ayant une dimension de pores comprise entre 100 et 2400 µm et une porosité comprise entre 25 % et 70 % en volume, **caractérisé par**
une étape consistant à appliquer sur l'objet multicouches une composition activant la croissance pour stimuler la croissance de nouveau tissu sur le site d'implantation et pour obtenir un objet implant poreux, cette composition activant la croissance renfermant un polymère biocompatible et une source de calcium, l'objet multicouches se décomposant dés l'implantation à une première vitesse pour former une ossature support pendant une période de temps prédéfinie, tandis que la composition activant la croissance, se décompose dés l'implantation à une seconde vitesse supérieure à la première pour stimuler la croissance de nouveau tissu sur l'implant.

9. Procédé conforme à la revendication 8,
selon lequel
l'objet implant poreux est chauffé pendant une durée et à une température efficace pour le recuire.

10. Procédé conforme à la revendication 8 ou 9,
selon lequel
un fin matériau flexible est enroulé autour de l'objet implant poreux et le vide est appliqué pour obtenir une enveloppe extérieure permettant de maintenir la composition biocompatible sur l'objet multicouches.

11. Procédé conforme à l'une des revendications 8 à 10,
selon lequel
le polymère biocompatible est choisi parmi l'acide polylactique, l'acide polyglycolique, les copolymères acide polylactique/acide polyglycolique, la polycaprolactone, et leurs combinaisons.

12. Procédé conforme à l'une des revendications 8 à 11,
selon lequel
le polymère biocompatible et la source de calcium sont mélangés selon des rapports compris entre 1:1 1 et 1:5.

13. Procédé conforme à l'une des revendications 8 à 12,
selon lequel
la viscosité de la composition polymère est comprise entre 100 et 500 centipoises à des températures comprises entre 80 et 100°C.

14. Utilisation d'une matrice polymère choisie parmi le polyméthyleméthacrylate, le polybutylènetéréphthalate, la polyéthyléthercétone et leurs combinaisons en tant que composition résorbable, et d'une composition activant la croissance renfermant un polymère biocompatible choisi parmi l'acide polylactique, l'acide polyglycolique, les copolymères acide polylactique/acide polyglycolique, la polycaprolactone, et leurs combinaisons, et d'une source de calcium, pour la préparation d'un implant biocompatible pour l'implantation chirurgicale, cette matrice ayant une dimension de pores comprise entre 100 et 2400 µm et une porosité comprise entre 25 % et 70 % en volume, et cet implant étant apte à activer une nouvelle croissance osseuse et de tissu pour l'implantation chirurgicale in vivo sur un site de remise en état choisi de façon à obtenir une base pour une nouvelle croissance osseuse et de tissu, la composition résorbable se décomposant dés l'implantation à une première vitesse pour former une ossature support pendant une période de temps prédéfinie, tandis que la composition activant la croissance se décompose à une seconde vitesse supérieure à la première vitesse pour stimuler la croissance de nouveau tissu sur l'implant.
